# FASCICULE DE BREVET EUROPEEN

(11) **EP 3 518 912 B1**
(45) Date de publication et mention de la délivrance du brevet: **30.12.2020**
(21) Numéro de dépôt: 17781051.2
(22) Date de dépôt: 28.09.2017
(51) Int. Cl.: A61K 31/155, A61K 31/192, C07C 279/26, C07C 323/62, A61K 9/00, A61P 1/16, A61P 3/06, A61P 3/10, A61P 3/04, A61P 9/00

(54) **SEL DE METFORMINE D'ELAFIBRANOR PRESENTANT UNE ACTIVITE DUALE POUR LE TRAITEMENT DE L'OBESITE ASSOCIEE A LA STEATO-HEPATITE NON ALCOOLIQUE (NASH) ET A L'HYPERTRIGLYCERIDEMIE**
DOPPELT WIRKENDES ELAFIBRANOR-METFORMIN-SALZ ZUR BEHANDLUNG VON ADIPOSITAS IM ZUSAMMENHANG MIT NICHTALKOHOLISCHER STEATOHEPATITIS (NASH) UND HYPERTRIGYCERIDÄMIE
DUAL-ACTION ELAFIBRANOR METFORMIN SALT FOR TREATING OBESITY ASSOCIATED WITH NON-ALCOHOLIC STEATOHEPATITIS (NASH) AND HYPERTRIGLYCERIDAEMIA

(30) Priorité: 30.09.2016 FR 1659435
(43) Date de publication de la demande: 07.08.2019
(73) Titulaire: Nashpharm, 06270 Villeneuve Loubet (FR)
(72) Inventeur: LARUELLE, Claude, 06270 Villeneuve Loubet (FR); BONNAFOUS, Ludovic, 06370 Mouans Sartoux (FR)
(74) Mandataire: Brizio Delaporte, Allison
(86) Numéro de dépôt international: PCT/EP2017/074703
(87) Numéro de publication internationale: WO 2018/060373

(56) Documents cités:
- EP-A1- 1 424 070
- EP-A1- 1 525 177
- EP-A1- 2 641 596
- WO-A1-2016/154258
- WO-A2-2009/017383
- WO-A2-2016/044433
- FR-A1- 2 774 591
- RATZIU VLAD ET AL: "Elafibranor, an Agonist of the Peroxisome Proliferator-Activated Receptor-[alpha] and -[delta], Induces Resolution of Nonalcoholic Steatohepatitis Without Fibrosis Worsening", GASTROENTEROLOGY, vol. 150, no. 5, 11 février 2016 (2016-02-11), page 1147, XP029522057, ISSN: 0016-5085, DOI: 10.1053/J.GASTRO.2016.01.038

## Description

### DOMAINE TECHNIQUE

La présente invention concerne les médicaments dérivés d'élafibranor.

La présente invention concerne plus particulièrement un dérivé de l'élafibranor présentant une activité duale pour le traitement de l'obésité associée à la stéato-hépatite non alcoolique (NASH) et à l'hypertriglycéridémie. Il s'agit d'un produit nouveau, le sel de metformine d'élafibranor (GFT505), un procédé de préparation dudit produit nouveau, et des compositions pharmaceutiques contenant, comme principe actif ledit produit nouveau.

L'invention concerne également des formulations pharmaceutiques sous diverses formes d'administration par voie entérale ou parentérale permettant de traiter ou de prévenir des maladies issues du syndrome métabolique telles que l'obésité, le surpoids, le diabète, la résistance à l'insuline, la dyslipidémie, les maladies hépatiques dont les stéatoses, fibroses ou cirrhoses, et les maladies cardiovasculaires qui en résultent. L'invention porte plus précisément sur des compositions pharmaceutiques permettant de traiter ou prévenir l'obésité associée à la stéato-hépatite non alcoolique (NASH).

### ETAT DE LA TECHNIQUE

L'obésité et les problèmes de poids sont un problème majeur dans les pays occidentaux. En 2012 en France, on dénombre 24,6 millions de personnes en surpoids, soit un tiers de la population. La moitié ont un problème de poids. L'obésité serait la cause de 13 % des décès en Europe. Il y a donc un besoin vital pour traiter ces maladies avec la recherche de molécules actives plus efficaces.

L'élafibranor décrit dans cette invention se réfère à la molécule expérimentale de la société Genfit. Elle peut apparaître citée sous son nom de code GFT505 ou GFT-505, développée initialement pour le traitement des maladies métaboliques incluant le diabète, la résistance à l'insuline, la dyslipidémie. Sa cible thérapeutique actuelle est le traitement des maladies hépatiques, en particulier la stéato-hépatite non alcoolique (NASH).

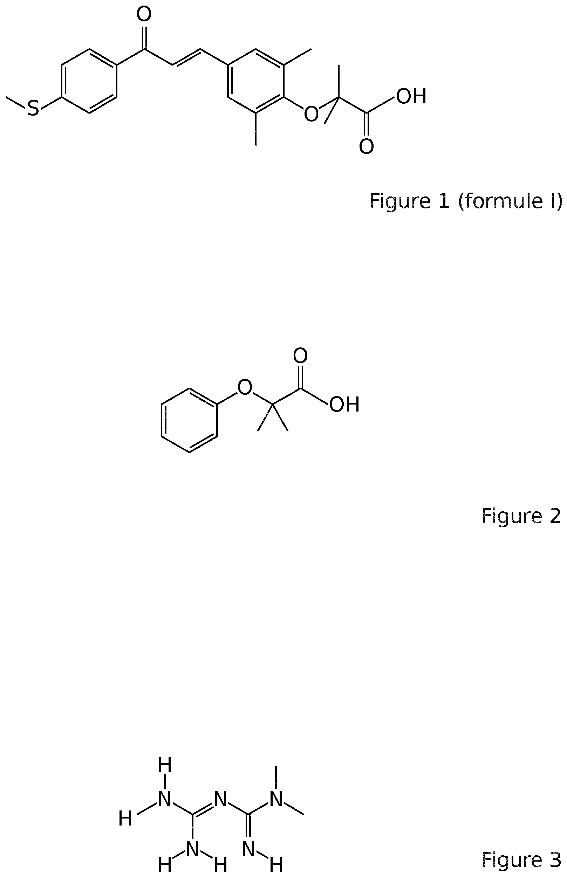

Son nom chimique est acide 2-[2,6 dimethyl-4-[3-[4-(methylthio)phenyl]-3-oxo-1(E)-propenyl]phenoxyl]-2-methylpropanoïque, de formule chimique C₂₂H₂₄O₄S et de poids moléculaire de 384,489 g/mol. Sa structure chimique de formule I est donnée dans la figure 1.

Le brevet EP 1525177 B1 de Genfit décrit l'utilisation, la préparation de molécules de la famille des 1,3-diphénylprop-2-en-1-one. L'élafibranor, appelé composé 29 dans ce document, est identifié par un spectre RMN (1H DMSO) avec les caractéristiques suivantes (δ ppm): 1.39 (s, 6H), 2.22 (s, 6H), 2.57 (s, 3H), 7.40 (d, J = 8.55Hz, 2H), 7.57 (s, 2H), 7.62 (d, J = 15.5Hz,1H), 7.83 (d, J = 15.5Hz, 1H), 8.10 (d, J = 8.55Hz, 2H), 12.97 (s, 1H) SM (ES- MS) : 383.3 (M-1).

Aucun élément publiquement disponible n'apporte plus de précisions sur l'identification physico-chimique de cette molécule qui comporte le même groupement acide propanoïque phénoxylé que les molécules de la famille des fibrates (figure 2).

Les molécules appartenant à la famille des fibrates sont connues pour leur faible solubilité aqueuse, et pour abaisser la concentration plasmatique des triglycérides et celle du cholestérol des lipoprotéines de très faible densité.

Les fibrates sont connus pour activer les récepteurs nucléaires appelés PPAR (peroxisome proliferator activating receptors), notamment les isoformes alpha, qui régulent la transcription des gènes impliqués dans le métabolisme des lipoprotéines riches en triglycérides et des HDL (« bon cholestérol »).

Les autres informations disponibles sur la molécule d'élafibranor concernent principalement les propriétés précliniques, cliniques et toxicologiques de cette molécule. A l'instar de fibrates, l'élafibranor y est identifié comme étant un co-activateur des récepteurs nucléaires PPARα/δ. Les essais cliniques montrent un très bon profil de tolérance de cette molécule, conforté notamment par des études toxicologiques à fortes doses chez l'animal incluant des études de carcinogénicité.

L'élafibranor (GFT05) présente des effets bénéfiques sur la stéato-hépatite non alcoolique NASH avec l'amélioration des marqueurs biochimiques de dysfonctionnement hépatique, comme les enzymes hépatiques : ALAT, ASAT, yGT, et ALP7.

Actuellement, il n'y a pas de descriptions approfondies des données physico-chimiques de l'élafibranor, qu'il s'agisse de la molécule chimique seule ou en tant que molécule active dans une composition pharmaceutique. Aucun sel physiologiquement acceptable n'est décrit dans des brevets ou publications scientifiques.

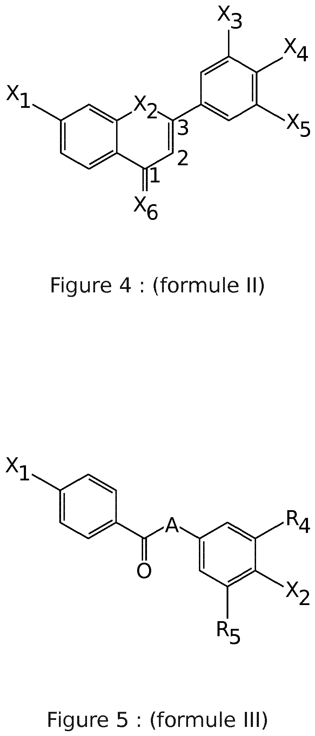

L'élafibranor (GFT505) est décrit depuis 2003 dans plusieurs brevets de la société Genfit qui couvrent toute application thérapeutique et, depuis 2009, sur une nouvelle application thérapeutique spécifique, en particulier pour le traitement de la stéato-hépatite non alcoolique NASH.
Les brevets EP1525177 et US7943661 portent sur une nouvelle famille de dérivés des chalcones. Ils décrivent le procédé de préparation et l'utilisation de dérivés de 1,3-diphénylprop-2-èn-1-one substitués de formule II ci-après (figure 4), dont fait partie la molécule élafibranor (composé 29 décrit dans la description, revendication 25), pour toute application thérapeutique, sans limitation à une maladie précise.
La seconde famille de brevets EP2504005, US8772342 et US9221751 porte sur des composés pour utilisation dans une méthode de traitement d'un trouble hépatique choisi dans le groupe constitué par une fibrose hépatique ou une stéatose hépatique. Notamment, la revendication 7 porte sur la molécule élafibranor pour une utilisation dans le traitement d'une fibrose hépatique ou d'une stéatose hépatique. Les revendications 9 et 10 portent sur une composition pharmaceutique comprenant un composé de formule III ci-dessous (figure 5) dans une méthode de traitement d'un trouble hépatique choisi dans le groupe constitué par une fibrose hépatique ou une stéatose hépatique.

Le brevet EP2504005B1 a fait l'objet d'une demande divisionnaire EP2641596A1 concernant les composés revendiqués dans le brevet EP2504005B1, mais utilisés cette fois-ci uniquement dans le cadre spécifique des maladies : cirrhose du foie, maladies liées à l'alcool, maladies hépatiques à médiation immunitaire.

D'autres brevets traitant de l'élafibranor sont à noter. Le brevet US7566737B porte sur une composition pharmaceutique comprenant une association entre un dérivé de 1,3-diphénylprop-2-èn-1-one substitué de formule II, y compris la molécule élafibranor, et un autre ingrédient ayant une activité thérapeutique.

Le brevet US8895619 B traite d'une méthode de traitement d'une fibrose hépatique par l'administration de la molécule Elafibranor (revendications 1-7, 10-11) et notamment pour traiter la cirrhose (revendications 8-9).

La demande US2016/0051501 porte sur une méthode de traitement d'une maladie du foie virale ou liée à l'alcool ou immunitaire par un composé de formule III (figure 5).

L'élafibranor n'est pas cité dans d'autres brevets. Seuls des résultats d'études apparaissent dans plusieurs articles dont les premiers sont publiés dès 2007 (Fruchart, Am J Cardiol 2007; 100[suppl]:41N-46N ; en 2013: Fruchart Cardiovascular Diabetology 2013, 12:82).

Le poster « The hepatic and extra-hepatic profile of resolution of steatohepatitis induced by GFT-505 (élafibranor)" de Sanyal AJ et al., traite des résultats d'une étude de phase 2b (Golden505) mettant en avant une dose journalière de 80 ou 120 mg en élafibranor administrée sur 270 patients NASH (3 groupes dont des diabétiques et non diabétiques). Il n'y a aucune précision sur la composition pharmaceutique des gélules dosées à 40 mg utilisées pour cette étude ni sur les caractéristiques physico-chimiques de l'élafibranor ou le rationnel concernant l'administration avant le petit déjeuner.

Les paramètres pharmacocinétiques dont le métabolisme ne sont pas publiquement disponibles pour l'élafibranor en dépit des études de phase 1 qui ont été réalisées. En 2012, dans l'étude de recherches de doses « Comparative Bioavailability - Gender Effect - Single and Multiple Ascending Dose Safety and Pharmacokinetic Study of GFT505*",* des modifications ont été apportées par la société Genfit dans les formulations d'élafibranor. Une étude de la biodisponibilité relative entre nouvelles et anciennes formulations a été menée, sur une gamme de dose atteignant 300 mg. Il n'y a pas de publication de résultats ni d'informations qui viennent justifier et étayer les raisons de ces travaux de formulation.

La metformine dont le nom chimique est 3-(diaminomethylidene)-1,1-dimethylguanidine présente une structure selon la figure 3 (formule I), et de formule chimique C₄H₁₁N₅. La substance est connue en tant que principe actif de médicament appartenant à la classe des biguanides antidiabétiques ayant une activité anti-hyper glycémique (Glucophage®, Glumetza® ...etc). La metformine est associée à une très faible incidence de l'acidose lactique. Il contribue à réduire les taux de cholestérol LDL et de triglycérides, et n'est pas associé à un gain de poids, et empêche les complications cardiovasculaires du diabète. La metformine n'est pas métabolisée et est excrétée inchangée par les reins.

La molécule active a pour poids moléculaire 129,16 g/mol et un point de fusion de 223-226 °C. Le sel de chlorhydrate de metformine est le plus utilisé en tant que principe actif dans les médicaments présents sur le marché tels que Glucophage®, du fait d'une très bonne solubilité dans l'eau (Log P -0.5 et pKa 12.4) et d'une stabilité chimique avérée même en conditions de température et d'humidité élevées (40°C/75%RH norme ICH).

La demanderesse a souhaité amélioré l'efficacité de l'élafibranor.

De manière surprenante, il a été trouvé que l'élafibranor sous forme de sel avec la metformine procure des effets avantageux différents de la somme de l'élafibranor et de la metformine pris individuellement. En effet, le sel de metformine permet une synergie d'action des principes actifs influençant notamment la biodisponibilité de ces derniers.

### BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les figures d'accompagnement suivantes dans lesquelles :
Figure 1: Formule chimique de l'Elafibranor ;
Figure 2 : Groupement chimique commun des fibrates et de l'Elafibranor
Figure 3 : Formule chimique de la metformine ;
Figure 4: Formule chimique dérivé du 1,3-diphénylprop-2-èn-1-one substitué comprenant l'Elafibranor.
Figure 5 : Formule générale d'un composé de la demande de brevet EP2504005 comprenant l'Elafibranor.
Figure 6 : Schéma de synthèse de l'Elafibranor (GFT505)
Figure 7A: Spectre RNM 1H Elafibranor (GFT505)
Figure 7B: UPLCMS Elafibranor (GFT505)
Figure 7C: Spectre UV Elafibranor (GFT505)
Figure 8A: spectre RNM 1H sel de metformine d'Elafibranor (GFT505)
Figure 8B : UPLCMS /spectre UV sel de metformine d'Elafibranor (GFT505)
Figure 9 : UPLCMS sel de metformine d'Elafibranor (GFT505) poudre après 14 jours sous forme poudre (groupe 1 lumière, groupe 2 protection lumière)

### EXPOSE DE L'INVENTION

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention concerne une composition comprenant comme principe actif, un sel pharmaceutiquement acceptable de metformine d'élafibranor (GFT505).

Avantageusement, l'invention concerne une composition comprenant au moins un principe actif caractérisé en ce que le au moins un principe actif comprend un sel de metformine-élafibranor

Avantageusement, la composition est destinée à traiter ou à prévenir des maladies issues du syndrome métabolique comprenant le diabète, l'obésité, les maladies hépatiques et cardio-vasculaires, la dyslipidémie.

Avantageusement, la composition est destinée à traiter ou à prévenir les maladies hépatiques choisies parmi les stéatoses hépatiques non alcoolique, les stéato-hépatites non alcooliques, les fibroses, les cirrhoses, les cancers.

Avantageusement, la composition est destinée à traiter ou à prévenir les maladies hépatiques caractérisé en ce que la maladie hépatique consiste en la stéatose hépatique non alcoolique (NAFLD).

Avantageusement, la composition est destinée à traiter ou à prévenir les maladies hépatiques caractérisé en ce que la maladie hépatique consiste en la stéato-hépatite non alcoolique (NASH).

Avantageusement, la composition est destinée à traiter ou à prévenir l'obésité.

Avantageusement, la composition est sous une forme adaptée pour une administration par voie orale.

Avantageusement, la composition est sous une forme adaptée pour une administration par voie parentérale

Avantageusement, la composition comprend tout au plus 500 mg de sel de metformine d'élafibranor.

Les modes d'administration par voie orale permettent une prise simple et rapide de la composition pharmaceutique.

Avantageusement, la composition est sous une forme adaptée pour une administration par voie intraveineuse.

Avantageusement, la composition est sous une forme adaptée pour une administration par voie sous -cutanée.

Avantageusement, la composition comporte au moins un excipient choisi parmi les liants, les agents désintégrants, les diluants, les lubrifiants, les agents tensioactifs, les agents tampons, les agents d'écoulement, les colorants, les arômes, les édulcorants, les solvants ou agents conservateurs.

L'invention concerne également un sel pharmaceutiquement acceptable de metformine d'élafibranor suivant la formule : C22H23O4S.C4H11 N5.

Avantageusement, la forme médicamenteuse de la composition consiste en une poudre pour solution injectable.

Avantageusement, la forme médicamenteuse consiste en une poudre pour suspension orale.

Le sel pharmaceutiquement acceptable d'élafibranor présente l'avantage d'avoir une meilleure solubilité dans l'eau comparativement à la forme base.

Avantageusement, la forme médicamenteuse consiste en la forme d'une solution injectable, d'un comprimé, d'un comprimé dispersible, d'un comprimé orodispersible, d'une gélule, d'un comprimé soluble, d'un lyophilisat, d'un comprimé effervescent, d'un comprimé à croquer, d'un comprimé à libération prolongée, d'un sachet.

Avantageusement, le profil de dissolution en milieux acide, dans l'eau et en milieux FaSSIF et FeSSIF simulant les prises de repas, le sel pharmaceutiquement acceptable de metformine d'élafibranor présente un pourcentage de dissolution supérieur à 90 % après 30 minutes.

L'invention concerne une utilisation d'une composition comprenant comme principe actif un sel pharmaceutiquement acceptable de metformine d'élafibranor (GFT505) pour l'obtention d'un médicament destiné à une utilisation dans traitement ou la prévention de maladies issues du syndrome métabolique, avec en particulier une activité duale pour le traitement de l'obésité associée à la stéato-hépatite non alcoolique (NASH) et à l'hypertriglycéridémie.

Dans un autre aspect, l'invention concerne la préparation du sel pharmaceutiquement acceptable de metformine d'élafibranor (GFT505) démontrant des propriétés physico-chimiques plus avantageuses que la forme base libre d'élafibranor, en particulier sur le plan de la solubilité et/ou de la stabilité.

### DESCRIPTION DETAILLEE

La présente invention se rapporte à l'utilisation d'un sel pharmaceutiquement acceptable de metformine d'élafibranor sous l'une de ses formes cristallines éventuellement polymorphes, ou bien amorphe, dans la préparation d'un médicament pour le traitement ou la prévention des maladies, en particulier les maladies liées au syndrome métabolique et présentant une activité duale quant à l'obésité et aux stéatoses hépatiques.

L'invention concerne également l'utilisation d'un sel pharmaceutiquement acceptable de metformine de l'acide 2-[2,6 dimethyl-4-[3-[4-(methylthio)phenyl]-3-oxo-1(E)-propenyl]phenoxyl]-2-methylpropanoïque, de formule chimique C₂₆H₃₄O₄N₅S pouvant être utilisés dans une composition pharmaceutique pour prévenir ou traiter les maladies, en particulier les maladies issues du syndrome métabolique telles que l'obésité, la résistance à l'insuline, les maladies du foie dont la stéato-hépatite non alcoolique NASH.

Le sel de metformine d'élafibranor est également dénommé sel de metformine d'élafibranorate.

La composition pharmaceutique de l'invention peut être administrée par voie entérale, par voie parentérale, topique ou sous cutanée. Selon un mode d'administration, la composition est administrée par voie entérale, tels que, par exemple, un comprimé, une gélule, une capsule molle, un lyophylisat, un comprimé dispersible, orodispersible, effervescent ou soluble, une solution orale, une poudre pour suspension orale.

Selon un mode préféré d'administration, la composition est administrée par voie orale sous la forme de comprimés, de gélules.

Selon un mode préféré d'administration, la composition est administrée par voie intraveineuse, par voie sous cutanée, sous la forme par exemple d'une solution injectable, d'une poudre pour solution injectable.

Les formulations destinées à être administrées par la voie intraveineuse ou orale contiennent un sel de metformine d'élafibranor cristallisé ou de structure amorphe afin d'optimiser le procédé de fabrication de la spécialité le cas échéant.

L'une des compositions pharmaceutiques préférées de l'invention est une poudre pour suspension orale ou pour préparation injectable soluble et stable en conditions normales de température et d'humidité.

La présente invention concerne donc en tant que produit nouveau le sel de metformine et de l'acide 2-[2,6 dimethyl-4-[3-[4-(methylthio)phenyl]-3-oxo-1(E)-propenyl]phenoxyl]-2-methylpropanoïque, mais également la préparation du sel de metformine et de cet acide 2-[2,6dimethyl-4-[3-[4-(methylthio)phenyl]-3-oxo-1(E)-propenyl]phenoxyl]-2-methylpropanoïque.

Cette préparation peut s'effectuer par un procédé de salification de l'acide 2-[2,6 dimethyl-4-[3-[4-(methylthio)phenyl]-3-oxo-1(E)-propenyl]phenoxyl]-2-methylpropanoïque par le diméthyl biguanide. Le procédé de préparation est donné dans les exemples ci-après.

### EXEMPLES

### EXEMPLE 1: Synthèse et caractérisation de l'élafibranor (GFT505)

La demanderesse a décidé de préparer des échantillons d'élafibranor pour évaluer la faisabilité des étapes de la synthèse de cette molécule et caractériser les propriétés physico-chimiques du produit obtenu. Le mode opératoire s'inspire des informations décrites dans le brevet EP 1525177 B1 pour la synthèse du composé 29. Les étapes sont reproduites à l'identique.

### Protocole expérimental

Le composé est synthétisé à partir de 1-[4-méthylthiophényl]-3-[3,5-diméthyl-tertiobutyloxycarbonyldiméthylméthyloxyphényl]prop-2-èn-1-one.

### Etape1 : 1-[4-méthylthiophényl]-(E)-3-(3,5-diméthyl-4-hydroxyphényl]prop-2-èn-1-one (intermédiaire 1)

La 4-méthylacétophénone (20 g, 0,12 mol, 1 eq) et le 3,5-diméthyl-4-hydroxybenzaldehyde (18 g, 0,12 mol, 1 eq) sont solubilisés dans 300mL de HCl 4N dans le dioxane. Le milieu réactionnel est agité 30 h puis les solvants sont évaporés. Purification par recristallisation à chaud dans 70 mL d'isopropanol et 12mL d'eau : 33 g (solide jaune, rendement : 92 %).
Formule brute : C₁₈H₁₈O₂S
ESI-MS m/z=299,18 [M+H]+
RMN ¹H DMSO-d6 δ ppm : 2.18 (s, 6H), 2.53 (s, 3H), 7.36 (d, J=8,5Hz, 2H), 7.47 (s, 2H), 7.57 (d, J= 15,5Hz, 1H), 7,69 (d, J= 15,5Hz, 1H), 8,05 (d, J= 8,5Hz, 2H), 8,93 (s, 1H)

### Etape 2 : 1-[4-méthylthiophényl]-(E)-3-(3,5-diméthyl-4-tertiobutylcarbonyldiméthylméthyloxyhényl]prop-2-èn-1-one (intermédiaire 2)

Le carbonate de césium (87 g, 0,134 mol, 4 eq) et l'iodure de tétrabutylammonium (12 g, 0,033 mol, 0,5 eq) sont ajoutés à une solution de l'intermédiaire 1 (20 g, 0,067 mol, 1 eq) dans 50mL d'un mélange DMSO/eau (3/2). Le milieu réactionnel est agité 30min à 80°C et le bromoisobutyrate de tertiobutyle (30 g, 0,134 mol, 2 eq) est ajouté. Puis 2 ajouts de 2 eq de bromoisobutyrate de tertiobutyle dilué à 50% dans le DMSO sont effectués chacun à 1 heure d'intervalle. Le milieu réactionnel est agité 2 jours à 80°C. Le milieu réactionnel est laissé refroidir à température ambiante puis 1,5L d'eau est ajouté et le produit est extrait avec du dichlorométhane (4 fois). La phase organique est séchée sur une cartouche séparatrice de phase et évaporée à sec. Purification sur gel de silice (cyclohexane/acétate d'éthyle : 95/5 à 80/20) : 18g (solide orange, rendement : 61%)
Formule brute : C₂₆H₃₂O₄S
ESI-MS m/z=441,33 [M+H]+
RMN ¹H DMSO-d6 δ ppm : 1.36 (s, 6H), 2.19 (s, 6H), 2.48 (broad peak, H2O + 9H), 2.54 (s, 3H), 7.38 (d, J=8,4Hz, 2H), 7.55 (s, 2H), 7.59 (d, J= 15,6Hz, 1H), 7.80 (d, J= 15,6Hz, 1H), 8.07 (d, J= 8,4Hz, 2H)

### Etape 3 : 1-[4-méthylthiophényl]-(E)-3-[3,5-diméthyl-4-carboxydiméthyiméthyioxyhényl]prop-2-èn-1-one

L'intermédiaire 2 (25 g, 0,057 mol, 1 eq) est solubilisé dans 50 mL de dichloromethane et 22 mL d'acide trifluoroacétique (5 eq, 0,284 mol) est ajouté doucement. Le milieu réactionnel est agité 3h30 à température ambiante puis les solvants sont évaporés à sec. Purification sur gel de silice (dichlorométhane/Méthanol : 100/0 -> 95/5) :13 g (solide jaune, rendement 60%).
Formule brute : C₂₂H₂₄O₄S
ESI-MS m/z=385,25 [M+H]+
RMN ¹H DMSO-d6 δ ppm : 1.36 (s, 6H), 2.19 (s, 6H), 2.54 (s, 3H), 7.37 (d, J=8,6Hz, 2H), 7.55 (s, 2H), 7.59 (d, J= 15,6Hz, 1H), 7.80 (d, J= 15,6Hz, 1H), 8.07 (d, J= 8,6Hz, 2H), 12.94 (s, 1H)

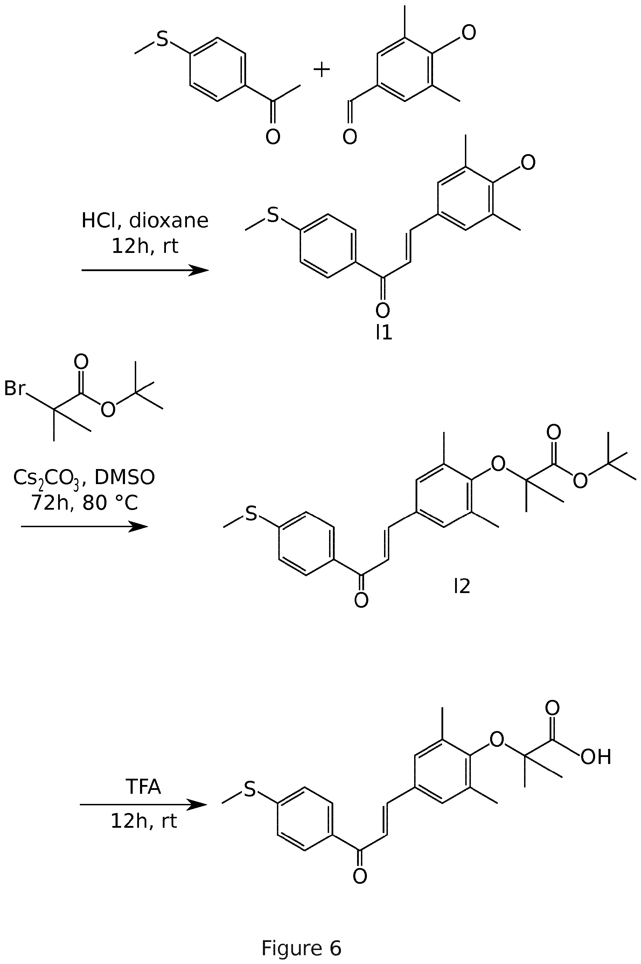

Le schéma réactionnel est donné dans la figure 6.

### Résultats

Les données d'analyses des 10,1 g du lot obtenu (EM0274L2) sont résumées ci-dessous :
- Masse moléculaire : 384,5 (masse exacte : 384,1) ;
- Spectre RMN 1H : Conforme à la structure, cf spectre en figure 6A ci-après.
- LCMS : TR=1,42mn, m/z: 385,00 = [M+H]+;
- Pureté : >98% (1H RMN et LCMS) ;
- Point de fusion : 144-145°C.

L'aspect du produit est une poudre solide jaune amorphe. Le produit présente une absorption notable dans le proche visible avec un Apex à environ 347 nm.

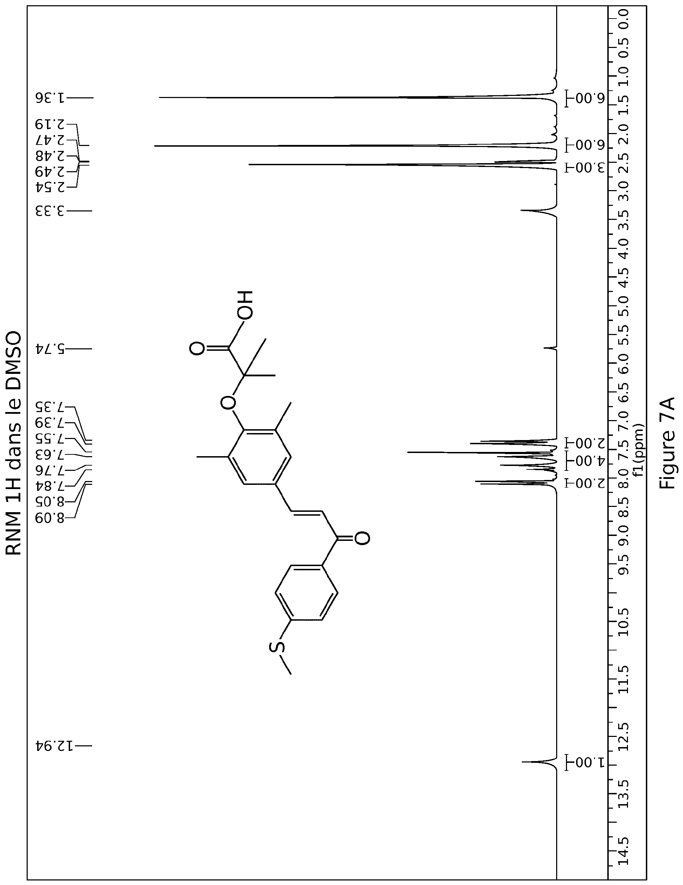

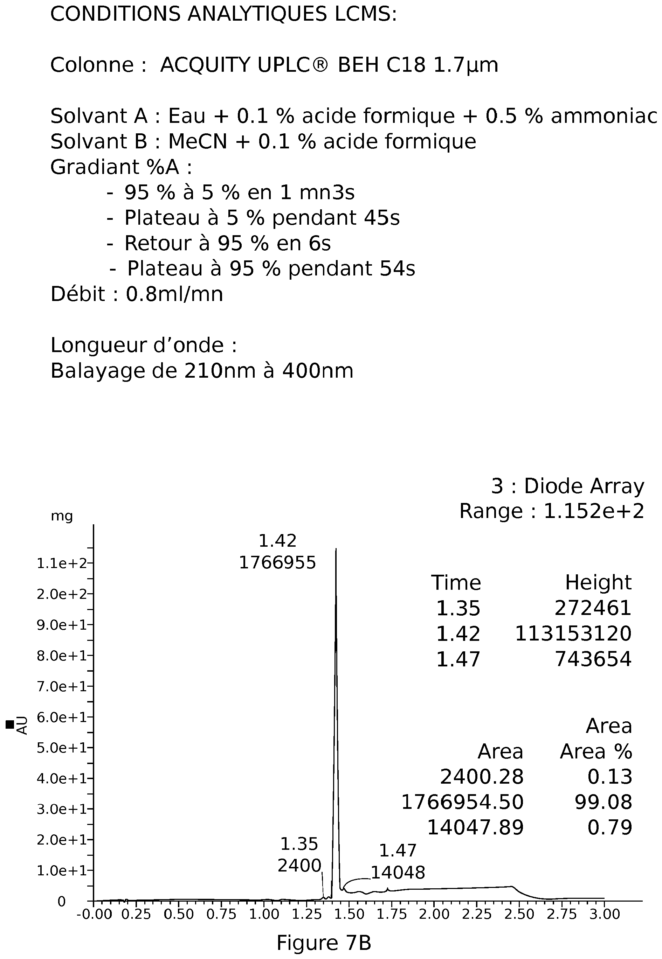

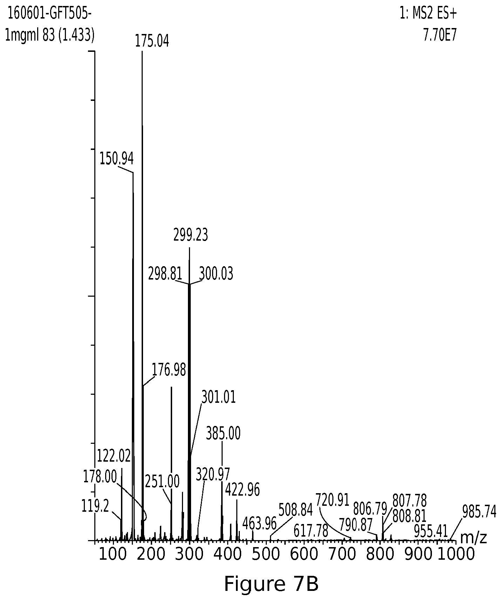

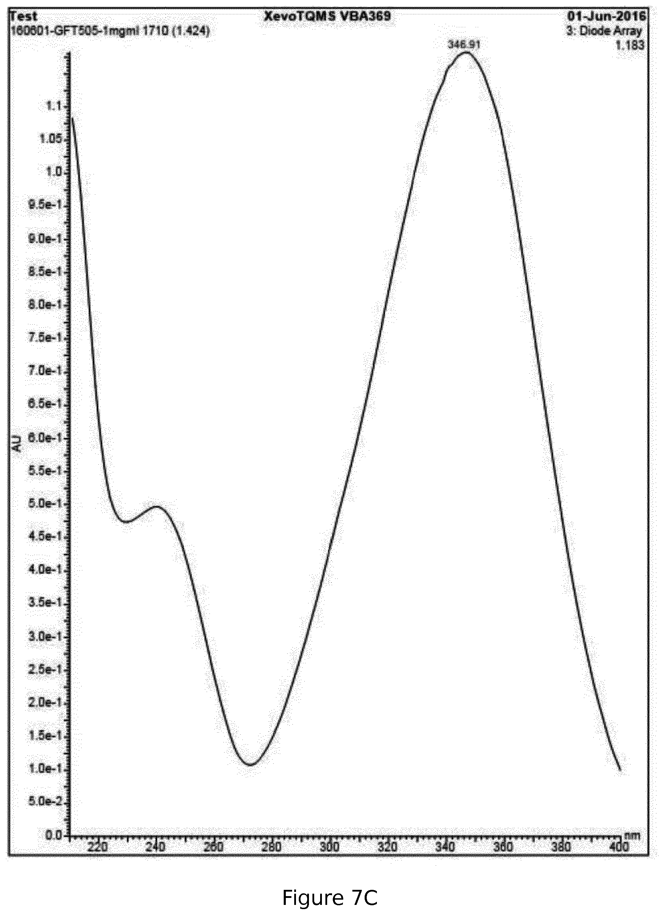

Les informations sont détaillées dans les figures 7 suivantes.
Le produit obtenu est conforme en termes de pureté chimique et démontre une absorption dans le proche visible qui nécessite que la stabilité chimique à la lumière et la phototoxicité doivent donc être vérifiées.

### EXEMPLE 2: Mesure de solubilité de l'élafibranor (GFT505)

Au cours d'une expérience réalisée pour l'invention, il a été montré que l'élafibranor (GFT505) affichait une structure chimique apparentée à la famille des fibrates (figure 2). L'élafibranor étant un acide carboxylique, la demanderesse a choisi de vérifier la solubilité aqueuse de cette molécule afin de statuer sur la faisabilité de développement de compositions pharmaceutiques en accord avec les attentes des patients, plus efficaces et mieux tolérées par les patients.

### Protocole expérimental

La solubilité thermodynamique de l'élafibranor base est étudiée sur une période de 24 heures et 72 heures dans divers tampons aqueux en présence ou non de tensioactifs. Le lot n°EM0274L2 est utilisé pour ces travaux. Le produit est dissous dans les solvants indiqués dans le tableau 1. Après 24h et 72h d'incubation à température ambiante (22 - 24°C), les solutions sont prélevées puis filtrées sur filtres en polycarbonate 0.2 µm dans des flacons pour analyses LCMS, et diluées une fois dans du DMSO avant agitation 2 minutes (Vortex ou sonification).

### Résultats

Les résultats de solubilité thermodynamiques sont donnés dans le tableau 1 ci-dessous :

**Tableau 1 : solubilité thermodynamique élafibranor en milieux aqueux.**

| Solvants | Solubilité après 24 h | | Solubilité après 72 h | |
|---|---|---|---|---|
| | µM | mg/mL | µM | mg/mL |
| Tampon pH 4,6 | 114 | 0,044 | 110 | 0,042 |
| Tampon pH 7,4 | 504 | 0,194 | 609 | 0,234 |
| Tampon pH 8,5 | 4419 | 1,701 | 4270 | 1,644 |
| Propylène Glycol | 33520 | 12,905 | 35081 | 13,506 |
| Polyéthylène Glycol 400 | 39022 | 15,023 | 40971 | 15,774 |

La solubilité de l'élafibranor est faible en milieu aqueux. Elle augmente en fonction du pH, passant de 114 à 4419 µM de pH 4,6 à 8,5. L'ajout de co-solvant comme le propylène glycol ou le PEG 400 permet d'améliorer significativement la solubilité de la molécule.

### EXEMPLE 3: Préparation de sels de metformine d'élafibranor (GFT505)

La demanderesse a préparé du sel de metformine d'élafibranor dans le but de déterminer ses caractéristiques et de les comparer à l'élafibranor sous forme de base libre, avec pour objectif la réalisation de compositions pharmaceutiques

### Protocole expérimental

Les lots de sels de metformine d'élafibranor sont élaborés à partir d'un lot d'élafibranor base libre.

1 g de GFT505 (2,6 mmol) et 104 mg de NaOH (1eq, 2,6mmol) sont mis en suspension dans 8ml d'isopropanol et 10ml de méthanol et chauffés à 65°C. Une solution de 431mg de Metformine.Cl (1eq, 2,6mmol) dans 2ml d'isopropanol est ajoutée et le milieu réactionnel jaune est agité pendant 30mn à 65°C. Après retour à température ambiante, la fine suspension est filtrée rapidement, lavée par 2x1ml d'isopropanol. Après environ 30mn, un solide jaune pâle commence à précipiter. Après 24h à température ambiante, le solide est filtré, lavé par 2x1ml d'isopropanol et séché pendant 24h à 45°C sous vide. Le résidu jaune est resolubilisé dans 10ml d'isopropanol et 6 ml de méthanol à 65°C. Le chauffage est arrêté est le milieu réactionnel limpide est laissé refroidir sous agitation. Un solide commence à précipiter vers 47°C. Après 24h, le solide est filtré, lavé par 2x1ml d'isopropanol et séché pendant 72h à 45°C sous vide (Lot CP0685L1, voir le Rapport n°1 du 30 aout 2016). Après 10 jours de séchage supplémentaire à 45°C sous vide poussé (<10-2 mbar), les traces de solvant détectées dans le lot précédent ont disparu (Lot CP0685L2).

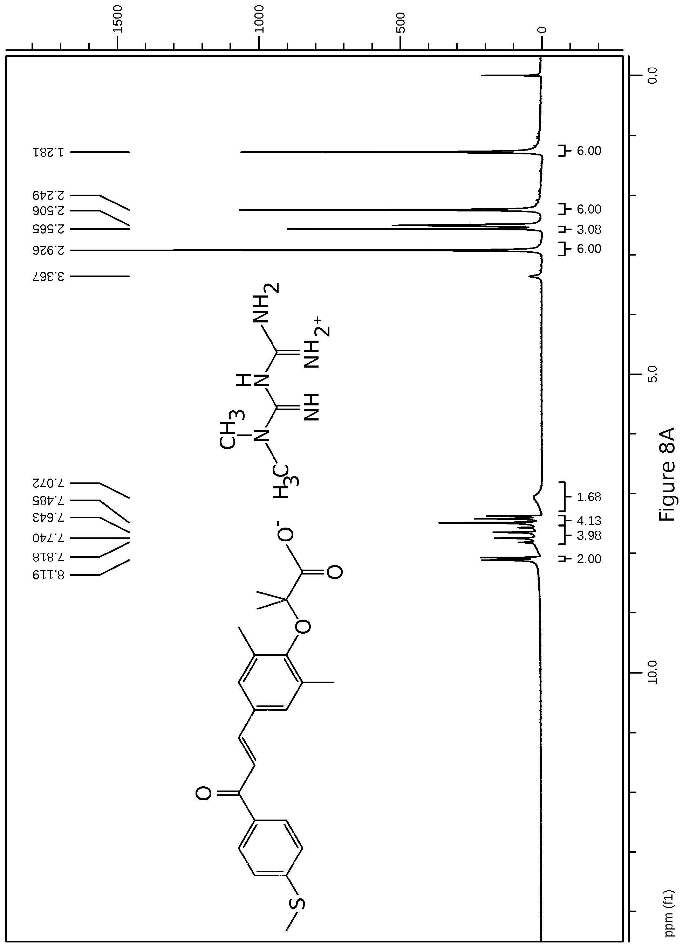

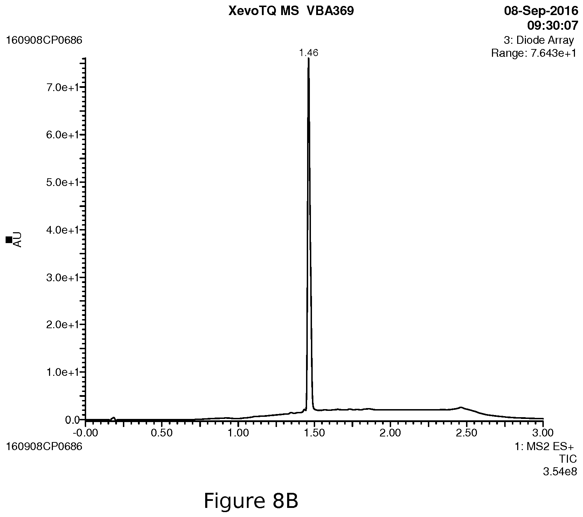

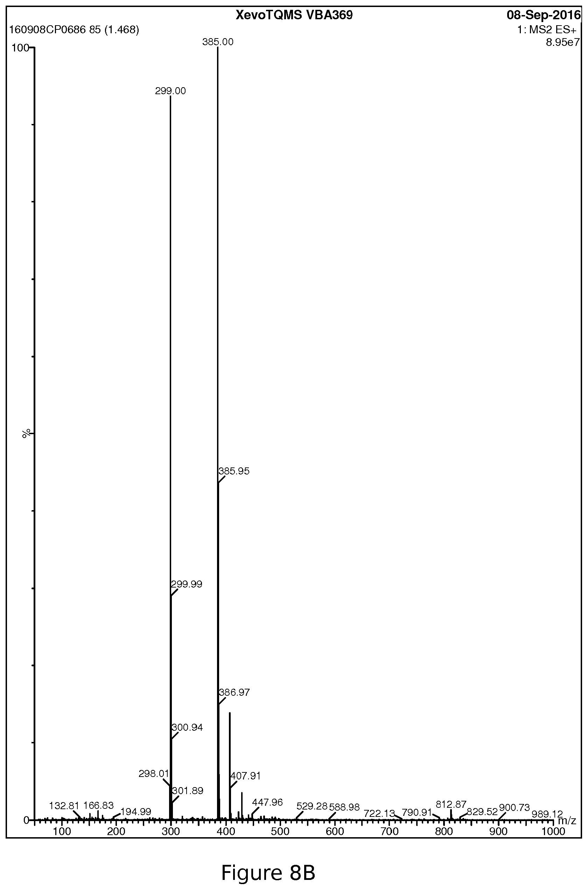

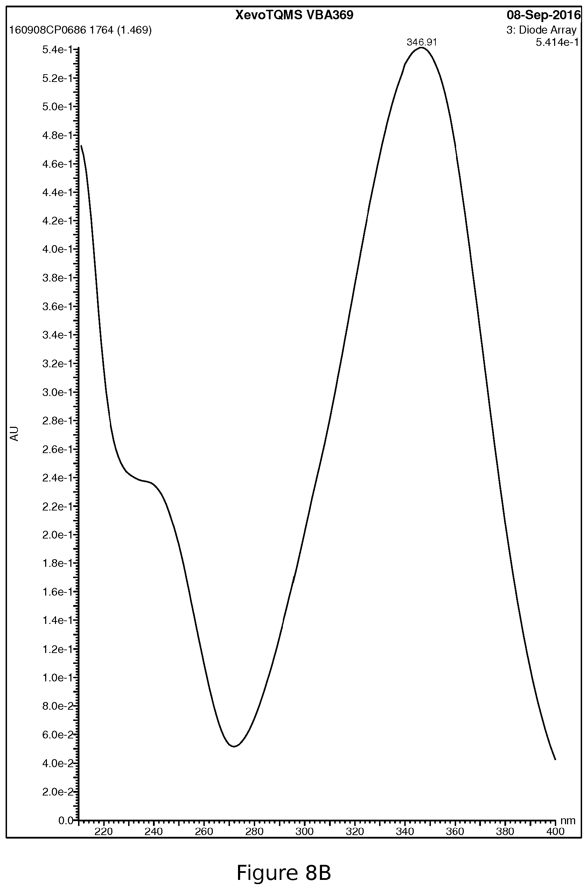

Les produits sont ensuite conservés au frais (2-8°C) et sous gaz inerte pour éviter toute dégradation. Une analyse du produit est réalisée, incluant identification et pureté chimique (voir figures 8).

### Résultats :

Les résultats sont les suivants :
Masse moléculaire : 513
Aspect : Solide jaune amorphe.
Point de fusion : 178/180°C.
TR=1,46mn, m/z: 385 = [M+H]+
Structure :

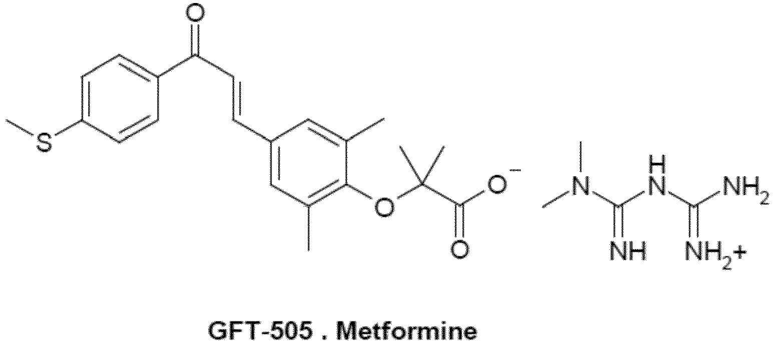

Autres données illustrées en figure 8.

### EXEMPLE 4: Test de solubilité du sel de metformine d'élafibranor (GFT505)

Cet exemple présente les caractéristiques de solubilité de différentes formes et sels d'élafibranor, en vue d'une administration par voie parentérale ou dans le cadre d'une composition entérale à libération rapide.

La cinétique de solubilisation est déterminée en milieux aqueux (eau et tampons pharmacopées), à température ambiante. Les résultats résumés sont indiqués dans le tableau ci-dessous.

**Tableau 2 : solubilité du sel de metformine d'élafibranor.**

| Solvants | Elafibranor (GFT505) sel de metformine | | Elafibranor (GFT505) Base libre amorphe | |
|---|---|---|---|---|
| | µM | mg/mL | µM | mg/mL |
| Tampon pH 4,6 | ND | ND | 114 | 0,044 |
| Tampon pH 7,4 | 12000 | 6 | 504 | 0,194 |
| Tampon pH 8,5 | ND | ND | 4419 | 1,701 |
| Propylène Glycol | - | - | 33520 | 12,905 |
| Polyéthylène Glycol 400 | - | - | 39022 | 15,023 |

Le sel de metformine d'élafibranor est environ plus de 20 fois plus soluble que l'élafibranor sous sa forme base libre.

### EXEMPLE 5: Stabilité de l'élafibranor et de l'un de ses sels.

La demanderesse a décidé de vérifier la stabilité de l'élafibranor et de ses sels de metformine après exposition à la lumière et à la température.

### Protocole expérimental

Des échantillons ont été préparés sous forme de poudre seule et de solutions aqueuses pour les échantillons suivants : élafibranor, élafibranor metformine.

La stabilité est mesurée sur une période de 7 à 14 jours par UPLCMS, avec calcul du taux de recouvrement du pic de l'élafibranor par rapport à la valeur initiale et mesure de son indice de pureté. Les produits sont exposés à la lumière du jour et à température ambiante.

Les échantillons références sont quant à eux stockés au frais (2-8°C), protégés de la lumière par un papier aluminium et sous gaz inerte pour le produit solide.

### Résultats

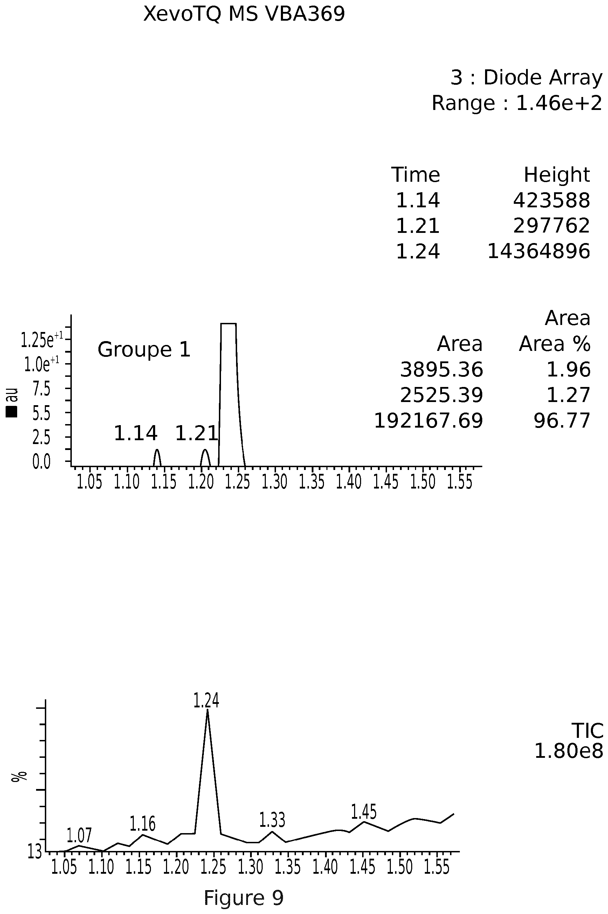

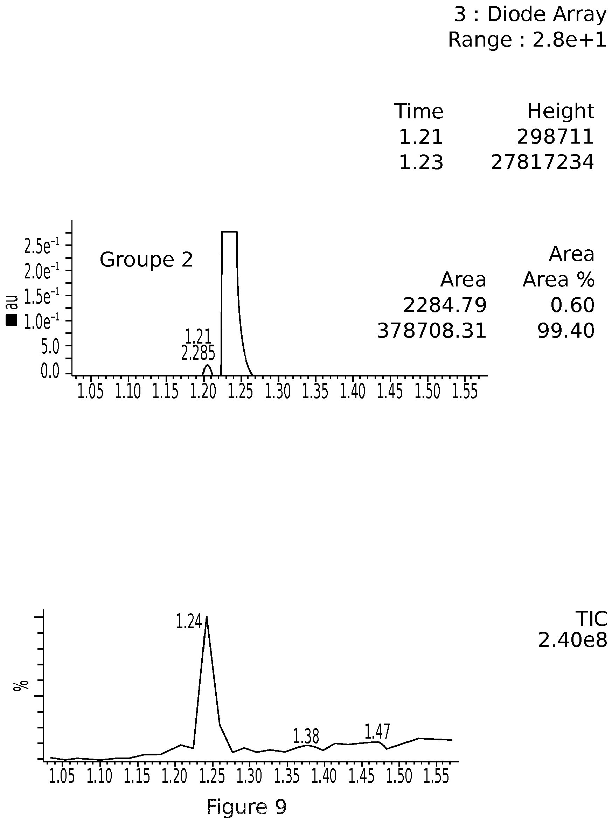

Les résultats démontrent que l'élafibranor base libre est plus sensible à la lumière (photosensibilité), que ce soit sous forme poudre ou bien en solution comparativement au sel. Le sel de metformine d'élafibranor, présente un changement notable de coloration de la poudre de jaune en jaune plus foncé suivant la même tendance que le produit sous sa forme de base libre. Néanmoins, le recouvrement après 14 jours de stockage est compris dans la norme 100 ± 5 %.: détection de produits de dégradation (<5 %). Figure 9 pour la LCMS (groupe 1 avec lumière, groupe 2 avec protection via aluminium pour les échantillons poudre)

La température n'a pas d'impact sur la stabilité. Les produits de dégradation à la lumière n'ont pas été identifiés.

## Revendications

1. Composition comprenant au moins un principe actif **caractérisé en ce que** le au moins un principe actif comprend un sel de metformine d'élafibranor.

2. Composition selon la revendication précédente pour son utilisation pour traiter ou prévenir des maladies issues du syndrome métabolique comprenant le diabète, l'obésité, les maladies hépatiques et cardio-vasculaires, la dyslipidémie.

3. Composition selon l'une quelconque des revendications précédentes pour son utilisation pour traiter ou prévenir les maladies hépatiques choisies parmi les stéatoses hépatiques non alcoolique, les stéato-hépatites non alcooliques, les fibroses, les cirrhoses, les cancers.

4. Composition selon la revendication précédente pour son utilisation pour traiter ou prévenir les maladies hépatiques **caractérisé en ce que** la maladie hépatique consiste en la stéatose hépatique non alcoolique (NAFLD).

5. Composition selon la revendication 3 pour son utilisation pour traiter ou prévenir les maladies hépatiques **caractérisé en ce que** la maladie hépatique consiste en la stéato-hépatite non alcoolique (NASH).

6. Composition selon la revendication 2 pour son utilisation pour traiter ou prévenir l'obésité.

7. Composition ou composition pour son utilisation selon l'une quelconque des revendications précédentes **caractérisée en ce qu'**elle est sous une forme adaptée pour une administration par voie orale.

8. Composition ou composition pour son utilisation selon l'une quelconque des revendications 1 à 6 **caractérisée en ce qu'**elle est sous une forme adaptée pour une administration par voie parentérale.

9. Composition ou composition pour son utilisation selon l'une quelconque des revendications 1 à 6 **caractérisée en ce qu'**elle est sous une forme adaptée pour une administration par voie intraveineuse.

10. Composition ou composition pour son utilisation selon l'une quelconque des revendications 1 à 6 **caractérisée en ce qu'**elle est sous une forme adaptée pour une administration par voie sous - cutanée.

11. Composition ou composition pour son utilisation selon l'une quelconque des revendications précédentes **caractérisée en ce qu'**elle comporte au moins un excipient choisi parmi les liants, les agents désintégrants, les diluants, les lubrifiants, les agents tensioactifs, les agents tampons, les agents d'écoulement, les colorants, les arômes, les édulcorants, les solvants ou agents conservateurs.

12. Sel pharmaceutiquement acceptable de metformine d'élafibranor suivant la formule C₂₂H₂₃O₄S.C₄H₁₁N₅:

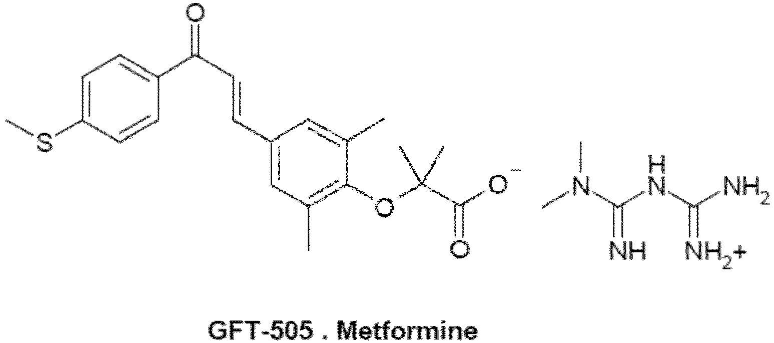

## Patentansprüche

1. Zusammensetzung, die mindestens einen Wirkstoff umfasst, **dadurch gekennzeichnet, dass** der mindestens eine Wirkstoff ein Elafibranor-Metforminsalz umfasst.

2. Zusammensetzung nach dem vorstehenden Anspruch zu deren Verwendung, um Erkrankungen zu behandeln oder vorzubeugen, die aus dem metabolischen Syndrom resultieren, die Diabetes, Adipositas, Leber- und Herz-Kreislauf-Erkrankungen, Dyslipidämie umfassen.

3. Zusammensetzung nach einem der vorstehenden Ansprüche zu deren Verwendung, um Lebererkrankungen zu behandeln oder vorzubeugen, ausgewählt aus nichtalkoholischen Lebersteatosen, nichtalkoholischen Steatohepatitiden, Fibrosen, Zirrhosen, Krebserkrankungen.

4. Zusammensetzung nach dem vorstehenden Anspruch zu deren Verwendung, um Lebererkrankungen zu behandeln oder vorzubeugen, **dadurch gekennzeichnet, dass** die Lebererkrankung in der nichtalkoholischen Lebersteatose (NAFLD) besteht.

5. Zusammensetzung nach Anspruch 3 zu deren Verwendung, um Lebererkrankungen zu behandeln oder vorzubeugen, **dadurch gekennzeichnet, dass** die Lebererkrankung in der nichtalkoholischen Steatohepatitis (NASH) besteht.

6. Zusammensetzung nach Anspruch 2 zu deren Verwendung, um Adipositas zu behandeln oder vorzubeugen.

7. Zusammensetzung, oder Zusammensetzung zu deren Verwendung nach einem der vorstehenden Ansprüche, **dadurch gekennzeichnet, dass** sie in einer Form vorliegt, die für eine Verabreichung auf oralem Wege geeignet ist.

8. Zusammensetzung, oder Zusammensetzung zu deren Verwendung nach einem der Ansprüche 1 bis 6, **dadurch gekennzeichnet, dass** sie in einer Form vorliegt, die für eine Verabreichung auf parenteralem Wege geeignet ist.

9. Zusammensetzung, oder Zusammensetzung zu deren Verwendung nach einem der Ansprüche 1 bis 6, **dadurch gekennzeichnet, dass** sie in einer Form vorliegt, die für eine Verabreichung auf intravenösem Wege geeignet ist.

10. Zusammensetzung, oder Zusammensetzung zu deren Verwendung nach einem der Ansprüche 1 bis 6, **dadurch gekennzeichnet, dass** sie in einer Form vorliegt, die für eine Verabreichung auf subkutanem Wege geeignet ist.

11. Zusammensetzung, oder Zusammensetzung zu deren Verwendung nach einem der vorstehenden Ansprüche, **dadurch gekennzeichnet, dass** sie mindestens einen Hilfsstoff umfasst, ausgewählt aus Bindemitteln, Sprengmitteln, Verdünnungsmitteln, Schmiermitteln, Tensiden, Puffermitteln, Fließmitteln, Farbstoffen, Geschmackstoffen, Süßungsmitteln, Lösungsmitteln oder Konservierungsmitteln.

12. Pharmazeutisch verträgliches Elafibranor-Metforminsalz der Formel C₂₂H₂₃O₄S.C₄H₁₁N₅:

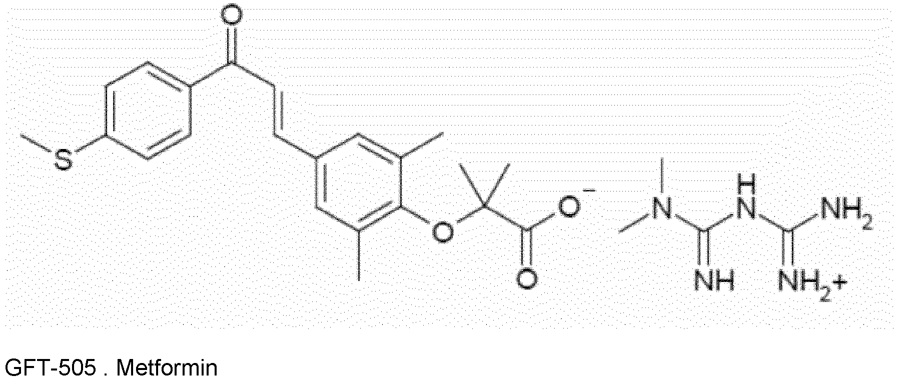

## Claims

1. Composition comprising at least one active ingredient, **characterised in that** the at least one active ingredient comprises an elafibranor metformin salt.

2. Composition according to the preceding claim for the use thereof for treating or preventing diseases coming from metabolic syndrome comprising diabetes, obesity, liver and cardiovascular diseases, dyslipidaemia.

3. Composition according to any one of the preceding claims for the use thereof for treating or preventing liver diseases selected from among non-alcoholic steatoses, non-alcoholic steatohepatitis, fibroses, cirrhosis, cancers.

4. Composition according to the preceding claim for the use thereof for treating or preventing liver diseases, **characterised in that** the liver disease consists of non-alcoholic fatty liver disease (NAFLD).

5. Composition according to claim 3 for the use thereof for treating or preventing liver diseases, **characterised in that** the liver disease consists of non-alcoholic steatohepatitis (NASH).

6. Composition according to claim 2 for the use thereof for treating or preventing obesity.

7. Composition or composition for the use thereof according to any one of the preceding claims, **characterised in that** it is in a form adapted for an oral administration.

8. Composition or composition for the use thereof according to any one of claims 1 to 6, **characterised in that** it is in a form adapted for a parenteral administration.

9. Composition or composition for the use thereof according to any one of claims 1 to 6, **characterised in that** it is in a form adapted for an intravenous administration.

10. Composition or composition for the use thereof according to any one of claims 1 to 6, **characterised in that** it is in a form adapted for a subcutaneous administration.

11. Composition or composition for the use thereof according to any one of the preceding claims, **characterised in that** it includes at least one excipient selected from among binding agents, disintegrating agents, dilutants, lubricants, surfactant agents, buffer agents, flow agents, colourants, flavours, sweeteners, solvents or preservative agents.

12. Pharmaceutically acceptable elafibranor metformin salt according to the formula C₂₂H₂₃O₄S.C₄H₁₁N₅:
